# EUROPÄISCHE PATENTSCHRIFT

(11) **EP 1 321 128 B1**
(45) Veröffentlichungstag und Bekanntmachung des Hinweises auf die Patenterteilung: **22.08.2007**
(21) Anmeldenummer: 02027911.3
(22) Anmeldetag: 13.12.2002
(51) Int. Cl.: A61K 8/02, A61K 8/86, A61Q 5/00

(54) **Schaumförmige Haarbehandlungsmittel mit einem Salzgehalt von 0,5 bis 20 Gew.%**
Hair mousse comprising salts in an amount of from 0,5 to 20%
Mousse pour cheveux à teneur en sels comprise entre 0,5 et 20%

(30) Priorität: 21.12.2001 DE 10163257
(43) Veröffentlichungstag der Anmeldung: 25.06.2003
(73) Patentinhaber: Henkel Kommanditgesellschaft auf Aktien, 40589 Düsseldorf (DE)
(72) Erfinder: Akram, Mustafa, 22457 Hamburg (DE); Emmerling, Winfried, 25436 Tornesch (DE); Hoepfner, Stefan, 22525 Hamburg (DE); Baumscheiper, Michael, 25436 Heidgraben (DE)

(56) Entgegenhaltungen:
- US-A- 5 496 538
- US-B1- 6 231 844
- PATENT ABSTRACTS OF JAPAN vol. 015, no. 472 (C-0889), 29. November 1991 (1991-11-29) & JP 03 200900 A (POLA CHEM IND INC), 2. September 1991 (1991-09-02)

## Beschreibung

Die vorliegende Erfindung betrifft die Verwendung spezieller Verbindungen zur Stabilisierung von salzhaltigen Schaumaerosolformulierungen sowie salzhaltige schaumförmige Aerosole, die diese Verbindungen enthalten.

Schaumaerosol-Zubereitungen werden in der Kosmetik seit langem angewandt. Sie kommen heutzutage in fast allen Bereichen der Kosmetik zum Einsatz, so sind beispielsweise Rasierschäume, Haarfestigungsmittel, Dauerwellmittel und Färbemittel häufig als Schaumaerosol formuliert.

Derartige Zubereitungen sind meist dünnflüssige O/W-Emulsionen, in deren Fettphase das Treibgas gelöst oder emulgiert ist. Wenn diese Mischung durch das Ventil gedrückt wird, versucht das Treibgas durch Verdunstung wieder in den energetisch günstigeren gasförmigen Zustand überzugehen. Da das Treibgas aber Bestandteil der internen Phase der Emulsion ist, kann das Gas nicht sofort entweichen und es bilden sich innerhalb der Emulsion Gasbläschen, so dass ein Schaum entsteht.

Häufig wurde in der Vergangenheit der Versuch unternommen, auch salzhaltige Emulsionen als Schaumaerosol zu formulieren. Dabei trat aber regelmäßig das Problem auf, dass die das Treibgas enthaltenden Wirkstoffemulsionen nicht lagerstabil waren, wenn zusätzlich Salze in der Formulierung enthalten sind. Häufig wurde eine Zersetzung der Emulsion beobachtet, so dass das Treibgas nach der Lagerung nicht mehr Bestandteil der Emulsion war, sondern als separate Phase in dem Behälter vorlag. Derartig zersetzte Formulierungen bilden während der Anwendung keinen akzeptablen Schaum aus.

Es wurde nunmehr überraschenderweise gefunden, dass durch den Einsatz spezieller Verbindungen derartige Lagerinstabilitäten bei salzhaltigen Schaumaerosolformulierungen vermieden werden können.

Ein erster Gegenstand der vorliegenden Erfindung ist daher die Verwendung von 0,1 bis 10,0 Gew % mindestens einer Verbindung der Formel (I)

R-O-(C₂H₄-O)ₓ-(C₃H₆-O)_{y}-H (I)

wobei R steht für einen linearen oder verzweigten Alkylrest mit 8 bis 30 C-Atomen, x steht für eine ganze Zahl von 0 bis 50 und y steht für eine ganze Zahl von 0 bis 25, mit der Maßgabe, dass mindestens eine der Variablen x oder y von null verschieden ist, zur Stabilisierung von salzhaltigen, kosmetischen Schaumaerosolformulierungen, mit einem Salzgehalt von 0,5 bis 20,0 Gew %.

Beim erfindungsgemäßen Einsatz der Verbindungen der Formel (I) konnte überraschenderweise häufig auf ein Schütteln der Dose während der Anwendung verzichtet werden.

Als bevorzugte Verbindungen der Formel (I) haben sich die Alkylenoxid-Anlagerungsprodukte an gesättigte Fettalkohole, die linear oder in 2-Stellung methylverzweigt sind, erwiesen. Der Alkylrest R weist bevorzugte 8 bis 22 Kohlenstoffatome auf. Solche Alkylreste sind beispielsweise die Reste 1-Octyl, 1-Decyl, 1-Lauryl, 1-Myristyl, 1-Cetyl und 1-Stearyl. Besonders bevorzugt sind 1-Lauryl, 1-Myristyl, 1-Cetyl und 1-Stearyl.

Bei den eingesetzten Verbindungen der Formel (I) mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Verbindungen der Formel (I), die Anlagerungsprodukte von Ethylen- und/oder Propylenoxid an Fettalkohole darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Erfindungsgemäß sind Verbindungen der Formel (I) bevorzugt, bei denen die Variablen x und y beide von null verschieden sind. Weiterhin sind Verbindungen der Formel (I) bevorzugt, bei denen die Variable x steht für eine Zahl von 1 bis 50 und die Variable y steht für eine Zahl von 1 bis 15.

Bevorzugte Verbindungen der Formel (I) sind beispielsweise die unter den INCI-Bezeichnungen PPG-2-Ceteareth-9, PPG-4-Ceteareth-12, PPG-10-Ceteareth-20, PPG-1-Ceteth-1, PPG-1-Ceteth-5, PPG-1-Ceteth-10, PPG-1-Ceteth-20, PPG-2-Ceteth-1, PPG-2-Ceteth-5, PPG-2-Ceteth-10, PPG-2-Ceteth-20, PPG-4-Ceteth-1, PPG-4-Ceteth-5, PPG-4-Ceteth-10, PPG-4-Ceteth-20, PPG-5-Ceteth-20, PPG-8-Ceteth-1, PPG-8-Ceteth-2, PPG-8-Ceteth-5, PPG-8-Ceteth-10, PPG-8-Ceteth-20, PPG-3-Laureth-9, PPG-4-Laureth-2, PPG-4-Laureth-5, PPG-4-Laureth-7, PPG-5-Laureth-5, PPG-6-Laureth-3, PPG-25-Laureth-25, PPG-3-Myreth-3, PPG-3-Myreth-11, PPG-9-Steareth-3 und PPG-23-Steareth-34 bekannten Verbindungen.

Besonders bevorzugte Verbindungen der Formel (I) sind erfindungsgemäß PPG-5-Ceteth-20, PPG-4-Ceteth-20, PPG-8-Ceteth-20 und PPG-4-Laureth-2.

Hinsichtlich der in den erfindungsgemäßen Schaumaerosolen eingesetzten Salzen unterliegt die vorliegende Erfindung keinen prinzipiellen Einschränkungen.

In einer ersten bevorzugten Ausführungsform werden die Verbindungen der Formel (I) in Schaumaerosolen vom Typ der Haarfärbe- oder -tönungsmittel verwendet. Die erfindungsgemäßen Haarfärbe- oder -tönungsmittel enthalten mindestens
- ein Salz eines Farbstoff(vorprodukt)es und/oder
- ein einfaches Salz, das als Beimischung aus dem Herstellprozess der Farbstoff(vorprodukt)e resultiert.

Erfindungsgemäß ganz besonders bevorzugte Farbstoff(vorprodukt)e, die in Salzform eingesetzt werden, sind p-Phenylendiamin, p-Toluylendiamin, 2-(β-Hydroxyethyl)-pphenylendiamin, 2,4,5,6-Tetraaminopyrimidin, 1-Methoxy-2-amino-4-(β-hydroxyethylamino)benzol, 3-Amino-2-methylamino-6-methoxypyridin, 2,4-Diaminophenoxyethanol, 1,3-Bis-(2',4'-diaminophenoxy)-propan, 5,6-Dihydroxyindolin, 4-(N-Ethyl, N-(β-hydroxyethyl)-amino)-1-(β-hydroxyethylamino)-2-nitrobenzol, 1,4-Bis-((β-Hydroxyethylamino)-2-nitrobenzol und 2-Amino-4,6-dinitrophenol sowie die unter den INCI-Deklarationen Basic Yellow-57, Basic Blue-99, Basic Red-76, Basic Brown-16 und Basic Brown-17 bekannten direktziehenden Farbstoffe.

Neben den in Form ihrer Salze vorliegenden Farbstoff(vorprodukt)en können die Mittel gemäß dieser Ausführungsform selbstverständlich weitere Farbstoff(vorprodukt)e enthalten.

Hinsichtlich der in den Mitteln dieser Ausführungsform einsetzbaren Farbstoffvorprodukte unterliegt die vorliegende Erfindung keinerlei Einschränkungen. Die erfindungsgemäßen Mittel können als Farbstoffvorprodukte
- Oxidationsfarbstoffvorprodukte vom Entwickler- und Kuppler-Typ, und
- Vorstufen naturanaloger Farbstoffe, wie Indol- und Indolin-Derivate,
sowie Mischungen von Vertretern dieser Gruppen enthalten.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 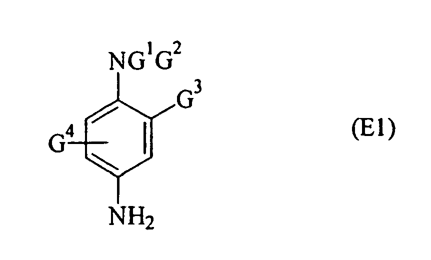 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, Iododer Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-pphenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-pphenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-pphenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 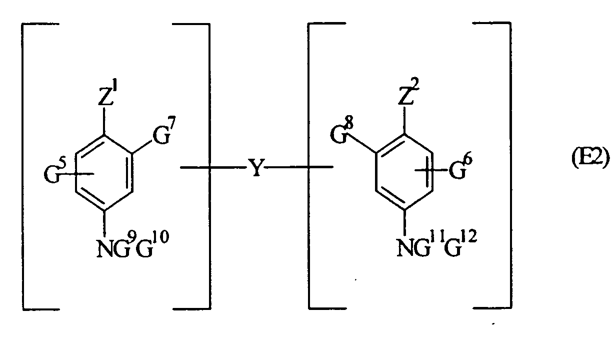 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, daß
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 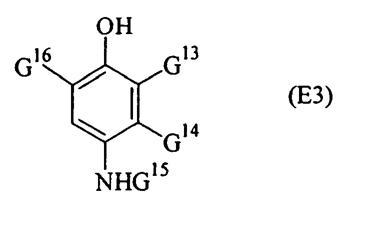 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁bis C₄)-Alkoxy-(C₁- bis C₄)-alkylrest, einen C₁- bis C₄-Aminoalkylrest oder einen C₁bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl- 1 -methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 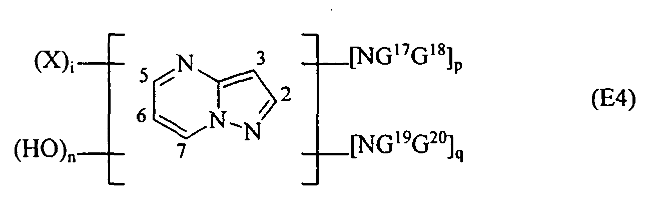 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂bis C₄-Polyhydroxyalkylrest einen (C₁- bis C₄)-Alkoxy-(C₁- bis C₄)-alkylrest, einen C₁bis C₄-Aminoalkylrest, der gegebenenfalls durch ein Acetyl-Ureid- oder einen SulfonylRest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird: 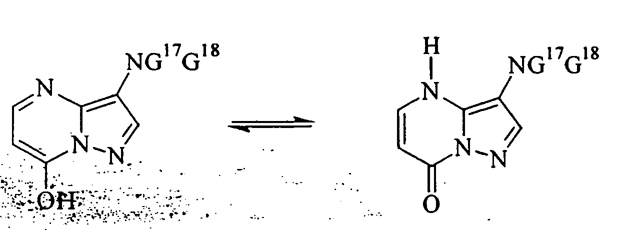 Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Neben den Entwicklerkomponenten können die erfindungsgemäßen Mittel weiterhin eine oder mehrere Kupplerkomponente enthalten. Als Kupplerkomponenten werden in der Regel m-Phenylendiaminderivate, Naphthole, Resorcin und Resorcinderivate, Pyrazolone und m-Aminophenolderivate verwendet.

Bevorzugte Kupplerkomponenten sind:
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (IIa), 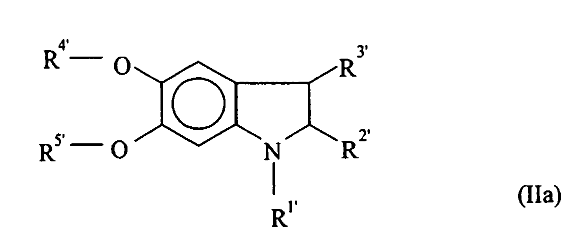 in der unabhängig voneinander
R^{1'} steht für Wasserstoff, eine C₁-C₄-Alkylgruppe oder eine C₁-C₄-Hydroxy-alkylgruppe,
R^{2'} steht für Wasserstoff oder eine -COOH-Gruppe, wobei die -COOH-Gruppe auch als Salz mit einem physiologisch verträglichen Kation vorliegen kann,
R^{3'} steht für Wasserstoff oder eine C₁-C₄-Alkylgruppe,
R^{4'} steht für Wasserstoff, eine C₁-C₄-Alkylgruppe oder eine Gruppe -CO-R^{6'}, in der
R^{6'} steht für eine C₁-C₄-Alkylgruppe, und
R^{5'} steht für eine der unter R^{4'} genannten Gruppen,
   sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb), 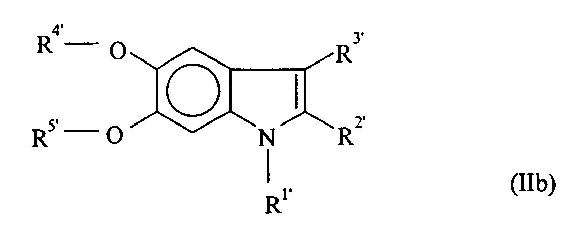 in der unabhängig voneinander
R^{1'} steht für Wasserstoff, eine C₁-C₄-Alkylgruppe oder eine C₁-C₄-Hydroxyalkylgruppe,
R^{2'} steht für Wasserstoff oder eine -COOH-Gruppe, wobei die -COOH-Gruppe auch als Salz mit einem physiologisch verträglichen Kation vorliegen kann,
R^{3'} steht für Wasserstoff oder eine C₁-C₄-Alkylgruppe,
R^{4'} steht für Wasserstoff, eine C₁-C₄-Alkylgruppe oder eine Gruppe -CO-R^{6'}, in der
R^{6'} steht für eine C₁-C₄-Alkylgruppe, und
R^{5'} steht für eine der unter R^{4'} genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Insbesondere bei der Verwendung von Farbstoff-Vorstufen vom Indolin- oder Indol-Typ hat es sich als vorteilhaft erwiesen, als Alkalisierungsmittel eine Aminosäure und/oder ein Oligopeptid einzusetzen.

Die einfachen Salze, die als Beimischung aus dem Herstellprozess resultieren, sind bevorzugter Weise ausgewählt aus den Chloriden, den Sulfaten und den Carbonaten von einwertigen oder zweiwertigen Kationen, ausgewählt aus den Alkalikationen, den Erdalkalikationen und dem Ammoniumkation. Natriumkationen sind besonders häufig als Beimischungen anzutreffen. Da die Schaumaerosolformulierung durch den erfindungsgemäßen Einsatz der Verbindungen der Formel (I) unempfindlich gegenüber derartigen Salzen wird, ist es nunmehr beispielsweise möglich, weniger aufwendig gereinigte Rohstoffe einzusetzen, die üblicherweise deutlich preisgünstiger sind.

Bevorzugte Beispiele für Farbstoffe, die häufig Salzbeimischungen aus dem Herstellprozess enthalten, sind 4[(3-Hydroxypropyl)amino]-3-nitrophenol und N'(β-Hydroxyethylamino)-2-nitro-4-aminobenzol.

In einer zweiten bevorzugten Ausführungsform der vorliegenden Erfindung enthalten die Schaumaerosolformulierung ein Salz einer aromatischen Säure. Derartige Salze werden in Schaumaerosolformulierungen beispielsweise als Konservierungsmittel eingesetzt. Besonders bevorzugte Salze einer aromatischen Säure sind die Salze der Benzoesäure, insbesondere das Natriumbenzoat, sowie die Salze der Derivate der Benzoesäure wie beispielsweise der p-Hydroxybenzoesäurealkylester. Ganz besonders bevorzugt sind die Natriumsalze des p-Hydroxybenzoesäurenmethylesters sowie des p-Hydroxybenzoesäurepropylesters.

In einer dritten bevorzugten Ausführungsform der vorliegenden Erfindung enthalten die Schaumaerosolformulierung ein Salz eines UV-Filters. Erfindungsgemäß sind die Salze der 2-Hydroxy-4-methoxybenzophenon-5-sulfonsäure, insbesondere die Natriumsalze, besonders bevorzugt.

Ein zweiter Gegenstand der vorliegenden Erfindung ist ein schaumförmiges Haarfärbe-oder Tönungsmittel, das es in einem kosmetisch akzeptablen Medium,
(A) 0,5 bis 20,0 Gew % eines Salzes,
(B) 0,1 bis 10,0 Gew % einer Verbindungen der Formel (I)

   R-O-(C₂H₄-O)ₓ-(C₃H₆-O)_{y}-H (I)

   wobei R steht für einen linearen oder verzweigten Alkylrest mit 8 bis 30 C-Atomen, x steht für eine ganze Zahl von 0 bis 50 und y steht für eine ganze Zahl von 0 bis 25, mit der Maßgabe, dass mindestens eine der Variablen x oder y von null verschieden ist, und
(C) mindestens ein Treibgas
enthält.

Die erfindungsgemäßen Mittel enthalten die Komponente (A) in einer Menge insbesondere von 0,5 bis 5,0 Gew.-%, jeweils bezogen auf das gesamte Mittel, eingesetzt. Die Bezugsgröße "gesamtes Mittel" versteht sich erfindungsgemäß inklusive der Treibgasphase.

Die Komponente (B) ist in den erfindungsgemäßen Mitteln bevorzugt in einer Menge insbesondere von 0,05 bis 5 Gew.-%, ganz besonders bevorzugt von 0,1 bis 1 Gew.-%, jeweils bezogen auf das gesamte Mittel, enthalten.

Erfindungsgemäße bevorzugte Treibgase sind ausgewählt aus den Kohlenwasserstoffen, den Fluor-Chlor-Kohlenwasserstoffen, den Fluorkohlenwasserstoffen sowie Dimethylether.

Erfindungsgemäß besonders bevorzugte Treibmittel sind Alkane mit 3 bis 5 Kohlenstoffatomen, wie Propan, n-Butan, iso-Butan, n-Pentan und iso-Pentan sowie deren Gemische; besonders bevorzugt sind n-Butan, iso-Butan und Propan sowie deren Mischungen. Ganz besonders bevorzugt ist der Einsatz von reinem Butan sowie von Mischungen des Butans mit bis zu 50Gew.-%Propan.

Die erfindungsgemäßen Mittel enthalten die Treibgase (Komponente (C)) in einer Menge von bis zu 20 Gew.-%, insbesondere von bis zu 10 Gew.-%, ganz besonders bevorzugt von bis zu 8 Gew.-%, jeweils bezogen auf das gesamte Mittel.

Weiterhin können die erfindungsgemäßen Mittel ein kationisches und/oder ein nichtionisches Tensid enthalten.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (IX)

   R³⁷CO-(OCH₂CHR³⁸)_{w}OR³⁹ Formel (IX),

   in der R³⁷CO für einen linearen oder verzweigten, gesättigten und/oder ungesättigten Acylrest mit 6 bis 22 Kohlenstoffatomen, R³⁸ für Wasserstoff oder Methyl, R³⁹ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Erfindungsgemäß bevorzugt sind kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Weiterhin können die erfindungsgemäßen Mittel mindestens einen Fettstoff enthalten. Unter Fettstoffen sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. In einer bevorzugten Ausführungsform beträgt die Menge 0,5 - 10 Gew.%, wobei ganz besonders vorteilhaft Mengen von 1-5 Gew.% sind.

Als Fettalkohole können eingesetzt werden gesättigte, ein- oder mehrfach ungesättigte, verzweigte oder unverzweigte Fettalkohole mit C₆- C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden. Die Fettalkohole werden in Mengen von 0,1 - 20 Gew.-%, bezogen auf die gesamte Zubereitung, bevorzugt in Mengen von 0,1 - 10 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, welche die Wirkung des erfindungsgemäßen Wirkstoffes steigern können, sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-nalkylether mit insgesamt zwischen 12 bis 36 C-Atomen, insbesondere 12 bis 24 C-Atomen, wie beispielsweise Di-n-octylether, Di-n-decylether, Di-n-nonylether, Di-nundecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykoldi-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Mono,- Di- und Trifettsäureester von gesättigten und/oder ungesättigten linearen und/oder verzweigten Fettsäuren mit Glycerin, wie beispielsweise Monomuls® 90-O18, Monomuls® 90-L12 oder Cutina® MD.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an Öl- und Fettkomponenten in den erfindungsgemäßen Mitteln beträgt üblicherweise 6 - 45 Gew.-%, bezogen auf das gesamte Mittel. Mengen von 10-35 Gew.-% sind erfindungsgemäß bevorzugt.

Weiterhin können die erfindungsgemäßen Mittel Hydroxycarbonsäureestern enthalten. Bevorzugte Hydroxycarbonsäureester sind Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol® der EniChem, Augusta Industriale. Die Einsatzmenge der Hydroxycarbonsäureester beträgt dabei 0,1 - 15 Gew.% bezogen auf das Mittel, bevorzugt 0,1 - 10 Gew.% und ganz besonders bevorzugt 0,1 - 5 Gew.%.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die folgenden Beispiele sollen den Gegenstand der vorliegenden Erfindung verdeutlichen ohne ihn jedoch zu beschränken.

### Beispiele

Die folgenden Angaben verstehen sich, soweit nichts anderes vermerkt ist, in Gewichtsprozent.

| Haartönungsmittel A | |
|---|---|
| Cetyltrimethylammoniumbromid | 1,5 |
| Cetylalkohol | 2,0 |
| Procetyl® AWS¹ | 1,0 |
| Monoethanolamin | 0,2 |
| Parfüm | 0,5 |
| p-Hydroxybenzoesäuremethylester | 0,2 |
| p-Hydroxybenzoesäurepropylester | 0,2 |
| Propan-Butan-Gemisch (50:50) | 10,00 |
| Arianor® Mahagoni² | 1,5 |
| Arianor® Straw Yellow³ | 0,3 |
| Arianor® Steel Blue⁴ | 0,5 |
| HC Red BN⁵ | 0,50 |
| 3-Amino-4-nitrophenol | 0,25 |
| Natriumsulfat | 2,0 |
| Wasser | ad 100 |

| | |
|---|---|
| ¹ Cetylalkohol mit ca. 20-EO- Einheiten und ca. 5 PO-Einheiten (INCI-Bezeichnung: PPG-5-Ceteth-20) (Croda) | |
| ² Basic Brown 16 (Warner Jenkinson) | |
| ³ Basic Yellow 57 (Warner Jenkinson) | |
| ⁴ Basic Blue 99 (Warner Jenkinson) | |
| ⁵ 4[(3-Hydroxypropyl)amino]-3-nitrophenol | |

Eine kastaniengroße Schaummenge wurde mittels einer Plastikbürste auf das mittelblonde Haar eines Probanden aufgebracht. Die Applikation wurde wiederholt bis die gesamte Haarpartie mit dem Farbschaum durchgekämmt war. Nach einer 20-minütigen Einwirkzeit wurde das Haar gespült und getrocknet. Das Haar wies einen brillanten Farbton in Rotkupfer mit schönem Glanz auf.

| Haartönungsmittel B | |
|---|---|
| Cetyltrimethylammoniumbromid | 1,5 |
| Cetylalkohol | 2,0 |
| Procetyl® AWS | 1,0 |
| Monoethanolamin | 0,2 |
| Parfüm | 0,5 |
| p-Hydroxybenzoesäuremethylester | 0,2 |
| p-Hydroxybenzoesäurepropylester | 0,2 |
| Propan-Butan-Gemisch (50:50) | 10,00 |
| 4-Hydroxyethylamino-3-nitrophenol | 0,5 |
| HC Red 3⁶ | 0,5 |
| HC Yellow No. 2⁷ | 0,3 |
| Kaliumchlorid | 3,0 |
| Wasser | ad 100 |

| | |
|---|---|
| ⁶ N(2'-Hydroxyethyl)-2-nitro-1,4-phenylendiamin ⁷ 1-(2'-Hydroxyethylamino)-2-nitrobenzol | |

Eine kastaniengroße Schaummenge wurde mittels einer Plastikbürste auf das hellblonde Haar eines Probanden aufgebracht. Die Applikation wurde wiederholt bis die gesamte Haarpartie mit dem Farbschaum durchgekämmt war. Nach einer 15-minütigen Einwirkzeit wurde das Haar gespült und getrocknet. Das Haar wies einen intensiven Goldton mit schönem Glanz auf.

| Haarfärbemittel C | |
|---|---|
| Cetyltrimethylammoniumbromid | 1,7 |
| Cetylalkohol | 2,2 |
| Procetyl® AWS | 1,5 |
| Monoethanolamin | 0,2 |
| Natriumsulfit | 0,1 |
| Parfüm | 0,5 |
| p-Hydroxybenzoesäuremethylester | 0,2 |
| p-Hydroxybenzoesäurepropylester | 0,2 |
| Propan-Butan-Gemisch (50:50) | 10,00 |
| p-Toluylendiamin-sulfat | 0,10 |
| 2-(β-Hydroxyethyl)-p-phenylendiamin-sulfat | 0,20 |
| 1,3-Dihydroxy-2-methylbenzol | 0,15 |
| 2-Amino-3-hydroxypyridin | 0,12 |
| 3-Methyl-4-aminophenol | 0,10 |
| 5,6-Dihydroxyindolin-hydrobromid | 0,20 |
| Wasser | ad 100 |

Eine kastaniengroße Schaummenge wurde auf eine Plastikbürste gedrückt und das teilergraute hellblonde Haar mit dem Produkt durchgekämmt. Die Applikation wurde wiederholt bis die gesamte Haarpartie mit dem Farbschaum durchgekämmt war. Nach einer 15-minütigen Einwirkzeit wurde das Haar gespült und getrocknet. Das Haar wies einen hellblonden Farbton mit guter Grauabdeckung auf.

| Färbemittel D | |
|---|---|
| Cetyltrimethylammoniumbromid | 1,5 |
| Cetylalkohol | 2,2 |
| Procetyl® AWS | 0,75 |
| Monoethanolamin | 0,2 |
| Natriumsulfit | 0,1 |
| Parfüm | 0,5 |
| p-Hydroxybenzoesäuremethylester | 0,2 |
| p-Hydroxybenzoesäurepropylester | 0,2 |
| Propan-Butan-Gemisch (50:50) | 10,00 |
| p-Phenylendiamin-hydrochlorid | 0,10 |
| 2-(β-Hydroxyethyl)-p-phenylendiamin-sulfat | 0,15 |
| 1,3-Dihydroxy-2-methylbenzol | 0,10 |
| 2-Amino-3-hydroxypyridin | 0,10 |
| 3-Methyl-4-aminophenol | 0,10 |
| 5,6-Dihydroxyindolin-hydrobromid | 0,10 |
| HC Red 3 | 0,20 |
| 3-Nitro-4-aminophenol | 0,10 |
| Wasser | ad 100 |

Eine kastaniengroße Schaummenge wurde auf eine Plastikbürste gedrückt und das teilergraute hellblonde Haar mit dem Produkt durchgekämmt. Die Applikation wurde wiederholt bis die gesamte Haarpartie mit dem Farbschaum durchgekämmt war. Nach einer 15-minütigen Einwirkzeit wurde das Haar gespült und getrocknet. Das Haar wies einen hellblond-kupfer Farbton mit guter Grauabdeckung auf.

## Patentansprüche

1. Verwendung von 0.1 bis 10 Gew.-% mindestens einer Verbindung der Formel (1)
R-O-(C₂H₄-O)ₓ-(C₃H₆-O)_{y}-H (I)
wobei R steht für einen linearen oder verzweigten Alkylrest mit 8 bis 30 C-Atomen, x steht für eine ganze Zahl von 0 bis 50 und y steht für eine ganze Zahl von 0 bis 25, mit der Maßgabe, dass mindestens eine der Variablen x oder y von null verschieden ist, zur Stabilisierung von salzhaltigen, kosmetischen Schaumaerosolformulierungen mit einem Salzgehalt von 0.5 bis 20,0 Gew.-%.

2. Verwendung nach Anspruch 1, **dadurch gekennzeichnet, dass** die Variablen x und y beide von null verschieden sind.

3. Verwendung nach einem der Ansprüche 1 oder 2, **dadurch gekennzeichnet, dass** die Variable x steht für eine Zahl von 1 bis 50 und die Variable y steht für eine Zahl von 1 bis 15.

4. Verwendung nach einem der Ansprüche 1 bis 3, **dadurch gekennzeichnet, dass** die Schaumacrosolformulierung Salze eines Farbstoff(vorprodukte)s enthält.

5. Verwendung nach einem der Ansprüche 1 bis 4, **dadurch gekennzeichnet, dass** die Schaumacrosolformulierung Chloride. Sulfate oder Carbonate von einwertigen oder zweiwertigen Kationen, ausgewählt aus den Alkalikationen, den Erdalkalikationen und dem Ammoniumkation, enthält.

6. Verwendung nach einem der Ansprüche 1 bis 5, **dadurch gekennzeichnet, dass** die Schaumaerosolformulierung ein Salz einer aromatischen Säure enthält.

7. Verwendung nach Anspruch 6, **dadurch gekennzeichnet, dass** es ein Salz der Benzoesäure oder eines Benzoesäurederivates ist.

8. Verwendung nach einem der Ansprüche 1 bis 7, **dadurch gekennzeichnet, dass** die Schaumaerosolformulierung ein Salz eines UV-Filters enthält.

9. Verwendung nach Anspruch 8, **dadurch gekennzeichnet, dass** das Salz ein Salz der 2-Hydroxy-4-methoxybenzophenon-5-sulfonsäure ist.

10. Schaumförmiges Haarfärbe- oder -tönungsmittel, **dadurch gekennzeichnet dass** es in einem kosmetisch akzeptablen Medium.
(A) 0.5 bis 20,0 Gew.-%, bezogen auf das gesamte Mittel, eines Salzes,
(B) 0,1 bis 10 Gew.-%, bezogen auf das gesamte Mittel, einer Verbindungen der Formel (I)
R-O-(C₂H₄-O)ₓ(C₃H₆-O)_{y}-H (I)
wobei R steht für einen linearen oder verzweigten Alkylrest mit 8 bis 30 C-Atomen, x steht für eine ganze Zahl von 0 bis 50 und y steht für eine ganze Zahl von 0 bis 25, mit der Maßgabe, dass mindestens eine der Variablen x oder y von null verschieden ist, und
(C) mindestens ein Treibgas
enthält.

11. Mittel nach Anspruch 10, **dadurch gekennzeichnet, dass** das Treibgas ausgewählt ist aus den Kohlenwasserstoffen, den Fluor-Chlor-Kohlenwasserstoffen, den Fluor-Kohlenwasserstoffen sowie Dimethylether.

12. Mittel nach Anspruch 11, **dadurch gekennzeichnet, dass** das Treibgas ausgewählt ist aus Propan, n-Butan und iso-Butan sowie deren Mischungen.

13. Mittel nach einem der Ansprüche 10 bis 12, **dadurch gekennzeichnet, dass** es weiterhin ein kationisches and/oder ein nichtionisches Tensid enthält.

14. Mittel nach einem der Anspruche 10 bis 13, **dadurch gekennzeichnet, dass** es mindestens ein Salz eines Farbstoff(vorprodukt)es enthält.

15. Mittel nach einem der Anspruche 10 bis 14, **dadurch gekennzeichnet, dass** es mindestens ein Salz einer aromatischen Säure enthält.

16. Mittel nach einem der Ansprüche 10 bis 15, **dadurch gekennzeichnet, dass** es mindestens ein Salz eines UV-Filters enthält.

17. Mittel nach einem der Ansprüche 10 bis 16, **dadurch gekennzeichnet, dass** es die Komponente (B) in einer Menge von 0.1 bis 1 Gew.-%, bezogen auf das gesamte Mittel, enthält.

18. Mittel nach einem der Ansprüche 10 bis 17, **dadurch gekennzeichnet, dass** es die Komponente (C) in einer Menge von bis zu 20 Gew.-% insbesondere von bis zu 10 Gew.-%, ganz besonders bevorzugt von bis zu 8 Gew.-%, jeweils bezogen auf das gesamte Mittel, enthält.

## Claims

1. Use of 0.1% to 10% by weight of at least one compound of the formula (I)
R-O-(C₂H₄-O)ₓ-(C₃H₆-O)_{y}-H (I)
where R is a linear or branched alkyl radical having 8 to 30 C atoms, x is an integer from 0 to 50 and y is an integer from 0 to 25, with the proviso that at least one of the variables, x or y, is other than zero, for stabilizing salt-containing cosmetic foam aerosol formulations having a salt content of 0.5% to 20.0% by weight.

2. Use according to Claim 1, **characterized in that** the variables x and y are both other than zero.

3. Use according to either of Claims 1 and 2, **characterized in that** the variable x is a number from 1 to 50 and the variable y is a number from 1 to 15.

4. Use according to any one of Claims 1 to 3, **characterized in that** the foam aerosol formulation comprises salts of a dye or dye precursor.

5. Use according to any one of Claims 1 to 4, **characterized in that** the foam aerosol formulation comprises chlorides, sulphates or carbonates of monovalent or divalent cations selected from the alkali metal cations, the alkaline earth metal cations and the ammonium cation.

6. Use according to any one of Claims 1 to 5, **characterized in that** the foam aerosol formulation comprises a salt of an aromatic acid.

7. Use according to Claim 6, **characterized in that** it is a salt of benzoic acid or of a benzoic acid derivative.

8. Use according to any one of Claims 1 to 7, **characterized in that** the foam aerosol formulation comprises a salt of a UV filter.

9. Use according to Claim 8, **characterized in that** the salt is a salt of 2-hydroxy-4-methoxybenzophenone-5-sulphonic acid.

10. Hair-colouring or hair-tinting composition in foam form, **characterized in that** it comprises, in a cosmetically acceptable medium,
(A) 0.5% to 20.0% by weight, based on the overall composition, of a salt,
(B) 0.1% to 10% by weight, based on the overall composition, of a compound of formula (I)
R-O-(C₂H₄-O)ₓ-(C₃H₆-O)_{y}-H (I)
where R is a linear or branched alkyl radical having 8 to 30 C atoms, x is an integer from 0 to 50 and y is an integer from 0 to 25, with the proviso that at least one of the variables, x or y, is other than zero, and
(C) at least one propellant gas.

11. Composition according to Claim 10, **characterized in that** the propellant gas is selected from the hydrocarbons, the hydrofluorochlorocarbons, the hydrofluorocarbons and dimethyl ether.

12. Composition according to Claim 11, **characterized in that** the propellant gas is selected from propane, n-butane and isobutane and mixtures thereof.

13. Composition according to any one of Claims 10 to 12, **characterized in that** it further comprises a cationic and/or a nonionic surfactant.

14. Composition according to any one of Claims 10 to 13, **characterized in that** it comprises at least one salt of a dye or dye precursor.

15. Composition according to any one of Claims 10 to 14, **characterized in that** it comprises at least one salt of an aromatic acid.

16. Composition according to any one of Claims 10 to 15, **characterized in that** it comprises at least one salt of a UV filter.

17. Composition according to any one of Claims 10 to 16, **characterized in that** it contains component (B) in an amount of 0.1% to 1% by weight, based on the overall composition.

18. Composition according to any one of Claims 10 to 17, **characterized in that** it contains component (C) in an amount of up to 20% by weight, more particularly of up to 10% by weight, with very particular preference of up to 8% by weight, based in each case on the overall composition.

## Revendications

1. Utilisation de 0,1 à 10% en poids d'au moins un composé de formule (I)
R-O-(C₂H₄-O)ₓ-(C₃H₆-O)_{y}-H (I)
où R représente un radical alkyle linéaire ou ramifié comprenant 8 à 30 atomes de carbone, x représente un nombre entier de 0 à 50 et y représente un nombre entier de 0 à 25, à condition qu'au moins une des variables x ou y soit différente de zéro, pour la stabilisation de formulations d'aérosol en mousse contenant du sel, cosmétiques, présentant une teneur en sel de 0,5 à 20,0% en poids.

2. Utilisation selon la revendication 1, **caractérisée en ce que** les variables x et y sont toutes les deux différentes de zéro.

3. Utilisation selon l'une quelconque des revendications 1 ou 2, **caractérisée en ce que** la variable x représente un nombre de 1 à 50 et la variable y représente un nombre de 1 à 15.

4. Utilisation selon l'une quelconque des revendications 1 à 3, **caractérisée en ce que** la formulation d'aérosol en mousse contient des sels d'un (précurseur de) colorant.

5. Utilisation selon l'une quelconque des revendications 1 à 4, **caractérisée en ce que** la formulation d'aérosol en mousse contient des chlorures, des sulfates ou des carbonates de cations monovalents ou divalents, choisis parmi les cations de métal alcalin, alcalino-terreux ou le cation d'ammonium.

6. Utilisation selon l'une quelconque des revendications 1 à 5, **caractérisée en ce que** la formulation d'aérosol en mousse contient un sel d'un acide aromatique.

7. Utilisation selon la revendication 6, **caractérisée en ce qu'**il s'agit d'un sel de l'acide benzoïque ou d'un dérivé de l'acide benzoïque.

8. Utilisation selon l'une quelconque des revendications 1 à 7, **caractérisée en ce que** la formulation d'aérosol en mousse contient un sel d'un filtre des UV.

9. Utilisation selon la revendication 8, **caractérisée en ce que** le sel est un sel de l'acide 2-hydroxy-4-méthoxybenzophénone-5-sulfonique.

10. Agent de teinture ou de coloration des cheveux sous forme de mousse, **caractérisé en ce qu'**il contient, dans un milieu cosmétiquement acceptable
(A) 0,5 à 20,0% en poids, par rapport à la totalité de l'agent, d'un sel,
(B) 0,1 à 10% en poids, par rapport à la totalité de l'agent, d'un composé de formule (I)
R-O-(C₂H₄-O)ₓ-(C₃H₆-O)_{y}-H (I)
où R représente un radical alkyle linéaire ou ramifié comprenant 8 à 30 atomes de carbone, x représente un nombre entier de 0 à 50 et y représente un nombre entier de 0 à 25, à condition qu'au moins une des variables x ou y soit différente de zéro, et
(C) au moins un agent propulseur.

11. Agent selon la revendication 10, **caractérisé en ce que** le gaz propulseur est choisi parmi les hydrocarbures, les hydrocarbures fluoro-chlorés, les hydrocarbures fluorés ainsi que le diméthyléther.

12. Agent selon la revendication 11, **caractérisé en ce que** le gaz propulseur est choisi parmi le propane, le n-butane et l'isobutane ainsi que leurs mélanges.

13. Agent selon l'une quelconque des revendications 10 à 12, **caractérisé en ce qu'**il contient en outre un agent tensioactif cationique et/ou non ionique.

14. Agent selon l'une quelconque des revendications 10 à 13, **caractérisé en ce qu'**il contient au moins un sel d'un (précurseur de) colorant.

15. Agent selon l'une quelconque des revendications 10 à 14, **caractérisé en ce qu'**il contient au moins un sel d'un acide aromatique.

16. Agent selon l'une quelconque des revendications 10 à 15, **caractérisé en ce qu'**il contient au moins un sel d'un filtre des UV.

17. Agent selon l'une quelconque des revendications 10 à 16, **caractérisé en ce qu'**il contient le composant (B) en une quantité de 0,1 à 1% en poids par rapport à la totalité de l'agent.

18. Agent selon l'une quelconque des revendications 10 à 17, **caractérisé en ce qu'**il contient le composant (C) en une quantité allant jusqu'à 20% en poids, en particulier jusqu'à 10% en poids, de manière tout particulièrement préférée jusqu'à 8% en poids, à chaque fois par rapport à la totalité de l'agent.
